# EUROPÄISCHE PATENTSCHRIFT

(11) **EP 3 769 700 B1**
(45) Veröffentlichungstag und Bekanntmachung des Hinweises auf die Patenterteilung: **13.12.2023**
(21) Anmeldenummer: 20195651.3
(22) Anmeldetag: 19.09.2018
(51) Int. Cl.: A61B 17/132, A61B 5/022, A61B 17/00, A61B 17/135, A61B 90/00

(54) **VORRICHTUNG ZUM STAUEN VON GEFÄSSEN**
DEVICE FOR STOWING CONTAINERS
DISPOSITIF POUR EMPILER DES RÉCIPIENTS

(30) Priorität: 20.09.2017 DE 102017121812
(43) Veröffentlichungstag der Anmeldung: 27.01.2021
(62) Teilanmeldung aus: 18786681.9
(73) Patentinhaber: Daisygrip GmbH, 18196 Kavelstorf (DE)
(72) Erfinder: ALTRICHTER, Konstantin, 22303 Hamburg (DE); HARTMANN, Karl, 18107 Elmenhorst Lichtenhagendorf (DE)
(74) Vertreter: Röthinger, Rainer

(56) Entgegenhaltungen:
- EP-A1- 2 745 785
- WO-A1-2014/191987
- WO-A1-2018/008607
- GB-A- 2 424 189
- GB-A- 2 540 197
- US-A1- 2004 133 219
- US-A1- 2008 262 535
- US-A1- 2010 063 206
- US-A1- 2011 247 179
- US-A1- 2016 113 660

## Beschreibung

Diese Erfindung bezieht sich auf eine hygienische Vorrichtung zum Stauen von Gefä-ßen (Gefäßstauer) mit einem Stauband, welches mit Hilfe eines Verschlusses zu einem geschlossenen Rundband verbunden wird. Der erfindungsgemäße Gefäßstauer, auch als Stauschlauch oder Venenstauer bekannt, findet in der Medizin Anwendung.

Gefäßstauer werden zum Erreichen einer Kompression angewendet, um beispielsweise in Blutgefäßen einen Staudruck zu erzeugen, z. B. bei einer Blutabnahme oder beim Legen von anderen Zugängen, oder um ein zeitweises Stauen eines Blutgefä-ßes für eine Blutdruckmessung zu ermöglichen.

Der alltägliche Gebrauch von Gefäßstauern bei der Routineblutentnahme führt ohne ordnungsgemäße Desinfektion unweigerlich zur Keimverschleppung. Allein in Deutschland haben nosokomiale (im Krankenhaus erworbene) Infektionen für 40.000 Patienten jährlich tödliche Folgen.

Vor jeder Blutentnahme wird ein Gefäßstauer angelegt. Das Spannen des Gefäßstauers führt zu einem Blutstau in den Venen, wodurch diese besser sichtbar werden und leichter mit einer Kanüle punktiert werden können. Eine solche Prozedur findet bei stationären Patienten oft mehrmals täglich statt. Nach jeder Anwendung ist eine Reinigung nötig.

Alle bisherigen Lösungen für Gefäßstauer bieten eine große Anlagerungsfläche für Keime. So werden beispielsweise Rundschläuche um den Arm eines Patienten gelegt und die Enden zusammengeknotet. Einige Lösungen erfordern auch ein präzises Einführen eines Bandes durch eine Lasche. Andere Lösungen verwenden einen mechanischen Verschluss, der ebenfalls einer aktiven Zusammenführung bedarf und durch die Steckverbindung viele Anlagerungsflächen für Keime bietet. Besonders die Beschaffenheit der bisherigen Bänder trägt zur Keimlast bei.

Die bisherigen Möglichkeiten, diese Gefäßstauer wieder in einen keimarmen Zustand zu überführen - zu reinigen, benötigen bis zu zwei Stunden im Sterilisationsgerät bzw. Autoklaven. Andere Möglichkeiten, wie Flüssigdesinfektionsmittel, erreichen auf Grund der Ausbildung der Gefäßstauer, deren Anlagerungsflächen und/oder des Bandmaterials, keinen adäquaten Reinigungszustand.

Die am weitesten verbreiteten Gefäßstauer, wie beispielsweise in US005535485A beschrieben, bestehen hauptsächlich aus einem elastischen frotteeartigen Band und einem zweiteiligen Verschlusselement. Aufgrund der mechanischen Konfiguration des Verschlusses hat die Oberfläche sehr viele Einkerbungen und bewegliche Teile, die Keimreservoire darstellen. Die bisherige Reinigungsvorschrift der Hersteller solcher Gefäßstauer zur Sicherstellung der Krankenhaushygiene sieht vor, dass diese Gefäßstauer im Autoklaviergerät behandelt werden. Dies erfordert eine etwa 2-stündige Prozedur aus Druck-, Dampf-, Wärme- und Strahlenbehandlung. Insbesondere das Stauband und die Verschlussteile stellen Keimreservoire dar, da beide eine große, raue und somit schwer zu reinigende Oberfläche besitzen. Dementsprechend müssen Gefäßstauer dieser Art im Autoklaviergerät behandelt werden, um eine Befreiung von Keimen zu ermöglichen. Makroskopische, unter anderem visuell sichtbare Verunreinigungen sind schwer bis gar nicht von dem Band zu lösen, Textilanteile und eine gewobene Struktur schließen andere Reinigungsmöglichkeiten, wie das Abwischen von Blutflecken, aus.

So offenbart das Patent DE 19650969 C1 einen Gefäßstauer mit einem Gurt und einer Schnalle, welche zwei Kupplungselemente aufweist. Ein erstes Kupplungselement weist ein hakenförmiges Verriegelungselement auf, das in ein zweites Kupplungselement lösbar einrastet. Die Kupplungselemente weisen übliche Klemmflächen zum Einklemmen des Gurts auf.

In dem Patent EP 633747 B1 wird eine Abschnürvorrichtung für Körperteile mit einem Abschnürband und einem Schlossgehäuse offenbart, bei der ein Ende des Abschnürbandes mit dem Schlossgehäuse verbunden ist und das freie Ende durch das Schlossgehäuse geführt wird. Eine Wippe dient zum Festklemmen des Bandes im Schlossgehäuse. Durch Druck auf ein Betätigungsteil wird ein langsames Entstauen ermöglicht.

Ein Tourniquet-Abbindesystem, wie in WO 2016/172599 A1 gezeigt, beschreibt eine Schnalle für eine Abschnürbinde, durch welche der Blutfluss durch Druck auf ein Blutgefäß unterbunden werden soll, um extremen Blutverlust zu vermeiden. Um diesen Zustand zu halten, werden u.a. magnetische Haltevorrichtungen genutzt. Durch das Drehen eines Stabes wird die mit diesem verbundene Abschnürbinde fest um den abzubindenden Körperteil geschnürt. Der Stab wird anschließend auf der Schnalle durch eine Lasche, Knöpfe oder eine magnetische Sicherung fixiert. Ein Spannen erfolgt dementsprechend unabhängig von der magnetischen Sicherung. Die Sicherung kann lediglich den vorher durch einen Stab aufgebauten Knebeldruck aufrechterhalten und ein Zurückdrehen des Stabes verhindern. Die Ausführung des Bandes und des Spann- und Haltemechanismus mit vielen Einzelteilen und versteckten Kanten stellen Keimreservoire dar, die einem keimarmen Arbeiten im klinischen Alltag entgegenstehen.

Ein erweitertes Tourniquet-Abbindesystem, wie in US 2014/0277103 A1 beschrieben, beschränkt sich weiterhin auf die Anwendung im Notfall. Auch ein zusätzlicher (Magnet-) Verschluss des Gurtes, in den wiederum nicht der Spannmechanismus integriert ist, führt nicht zu einer besseren Reinigbarkeit / Hygiene. Ein Gurt nur mit dem beschriebenen Verschluss kann nicht zum Blutstauen gespannt / gelockert werden. Er wäre wie bereits im Patent eigens beschrieben nicht als Tourniquet zu benutzen, sondern ein einfacher Gurt um Objekte zusammen zu halten. Dieser Umstand ist der Grundidee der Erfindung geschuldet, die sich auf die militärische Notfallversorgung konzentriert.

In CN 205831862 U ist ein elastisches Band an beiden Enden des Rundbandes mit jeweils einem Verschlussteil versehen. Die beiden Verschlussteile sind mit Magneten ausgerüstet. Der Anwender muss die Verschlussteile aktiv zusammenführen bzw. einhaken und anschließend die Magnete in eine Position bringen, in der die Magnetkräfte als Sicherung wirken können. Der Magnet wirkt auch hier nur zum Sichern in der Stauposition und unterstützt das Halten im gespannten Zustand. Die für das in-Position-Halten notwendigen hakenförmigen Hinterschneidungen bilden Anlagerungsmöglichkeiten für Keime, wodurch das Band mit den Verschlussteilen für eine Vor-Ort-Desinfektion kaum hygienegerecht aufbereitet werden kann.

In der Patentveröffentlichung CN 104207817 A wird eine Abschnürvorrichtung beschrieben, die auch aus Silikon sein kann, dessen Löcher durch den Gürtel-Mechanismus bedingten Verschluss jedoch ebenso gegen eine einfache Reinigung sprechen, wie die Verriegelung des Verschlusses, wodurch neue Öffnungen und Keimreservoire an dem Hebel/Bolzen entstehen. Weiterhin ist die Anwendung am Patienten zwingend mit zwei Händen durchzuführen.

Die US 2008/262535 A1 offenbart ein mechanisiertes Tourniquet, das ausgebildet ist, um arteriellen Blutverlust in einem verletzten Glied zu stoppen. Das Tourniquet wird wahlweise von einem Benutzer oder automatisch aktiviert. Das Tourniquet und dessen elektromechanische Spannvorrichtung werden automatisch mittels eines Bandspannsteuerungsmechanismus und eines durch ein Sicherheitssignal initialisierten Zeitgebers gesteuert.

Die WO 2018/008607 A1 offenbart ein hämostatisches Instrument. Das Instrument umfasst ein um ein Handgelenk zu wickelndes Band, ein Fixierungsmittel zum fixieren des Bandes in einem um das Handgelenk gewickelten Zustand, eine expandierende Einheit, die sich durch Einleiten von Luft ausdehnt und gegen die Stelle drückt, an der eine Blutung gestoppt werden soll.

Die GB 2 540 197 A offenbart ein Tourniquet, das einen aus Folienmaterial gebildeten Streifen umfasst. Der Streifen hat ein erstes Ende und ein zweites Ende. Der Streifen legt eine Öffnung zum ersten Ende hin fest und weist einen an das zweite Ende angrenzenden Abschnitt auf, der ausgebildet ist, um durch die Öffnung zu verlaufen. Das Tourniquet hat eine Vielzahl von Verriegelungselementen, die entlang des Abschnitts beabstandet sind. Die Verriegelungselemente erstrecken sich von dem Abschnitt und sind ausgebildet, um es dem Abschnitt zu ermöglichen, durch die Öffnung zu verlaufen. Die Verriegelungselemente sind ferner dazu ausgebildet, um dem aus der Öffnung herausstehenden Abschnitt, einen Widerstand entgegensetzen.

Die WO 2014/191987 A1 offenbart ein Druckgasbehälter und eine Durchflusssteuereinheit innerhalb eines starren Gehäuses einer Steuervorrichtung. Die Kontrollvorrichtung ist an einem aufblasbaren Gurt eines pneumatischen Tourniquets befestigt. Die Durchflusssteuerungseinheit ist ausgebildet, um wahlweise eine Dosis des aus dem Gasbehälter abgegebenen Druckgases in eine innere Gurtkammer zu leiten, um einen vorbestimmten Kammerdruck zum Aufbringen einer erforderlichen blutungsunterdrückenden Kraft auf eine Gliedmaße zu erreichen.

Die US 2011/247179 A1 offenbart eine Verschlussklinkenvorrichtung zum Festklemmen eines Bandes. Die Verschlussklinke umfasst ein erstes und ein zweites Klinkenelement, die in Eingriff stehen, um relativ zueinander zu schwenken.

Die US 2010/063206 offenbart ein Verfahren und eine Vorrichtung zum Fluorieren von Kunststoffen.

Die EP 2 745 785 offenbart ein ortsdefiniert aufklebbares Gefäßverschlusssystem zum Verschließen von geöffneten Blutgefäßen.

### Darstellung der Erfindung

Der Erfindung liegt die Aufgabe zugrunde, den Verschluss und insbesondere das Stauband eines Gefäßstauers so auszubilden, dass diese als hygienische Mehrweg-Venenstauer genutzt werden können, indem insgesamt eine einfachere Desinfektionsmöglichkeit, wie eine Sprüh-/Wischdesinfektion vor Ort, durchführbar ist, um so eine Keimverschleppung zu vermeiden. Darüber hinaus ist es die Aufgabe der Erfindung, dass die Verschlusselemente auch bei ungenauem Aneinanderfügen ihre Rastposition selbstständig finden. Das Zusammenfügen und Lösen des Verschlusses soll mit einer Hand erfolgen können.

Gelöst wird diese Aufgabe durch die in den Ansprüchen offenbarten Merkmale.

Die hier beschriebene Vorrichtung zum Stauen von Gefäßen umfasst ein Stauband, vorzugsweise mit einer Breite zwischen 0,5 und 50 cm, bevorzugt zwischen 1 und 10 cm für eine Ausführungsform, beispielsweise als Gefäßstauer, und zwischen 7 und 30 cm für eine weitere Ausführungsform, beispielsweise als Blutdruckmanschette, und einen Verschluss, welcher zwei Verschlusselemente enthält, wobei ein Ende des Staubandes fest mit dem einen Verschlusselement des Verschlusses verbindbar ist und das andere Verschlusselement verschiebbar auf dem Stauband anordenbar ist. Das Stauband und der Verschluss können aus einem Material mit einer glatten Oberfläche bestehen. Der Verschluss kann im geschlossenen Zustand ein glatter, quaderförmiger oder länglicher Körper sein, welcher außer den Zuführungen für das Stauband keine weiteren äußeren Öffnungen und durch bündig abschließende Kanten zwischen den Verschlusselementen keine äußeren Absätze aufweist. Unter bündig ist auch zu verstehen, dass die abschließenden Kanten weitestgehend bündig sind, d.h. sie können auch beispielsweise konisch oder konvex verlaufen. Das Stauband besteht aus einem textilfreien Material und das Stauband und optional der Verschluss sind durch Fluorierung von dessen Oberfläche mit einer Gleitbeschichtung versehen. Die Gleitbeschichtung kann die Oberfläche / die Oberflächen versiegeln.

Textilien erreichen ein Dehnungsmaximum aufgrund der Maschenanordnung. Dadurch treten schneller Abschnürungseffekte auf, die Weichteilquetschungen und Thrombosen nach sich ziehen. Ein Gummiband hat aufgrund der intermolekularen Effekte kein derartig ausgeprägtes Maximum, dadurch wird das Abschnüren verhindert und es sind weichere Einstellmöglichkeiten des Staudrucks realisierbar.

Materialien für den Verschluss könnte neben Kunststoff auch Aluminium, Edelstahl ö. Ä. sein. Das Stauband kann anstelle von Silikon auch aus einem anderen elastischen Material mit geschlossener Oberfläche bestehen, wie z. B. Latex, thermoplastische Elastomere, Teflon o. Ä..

In einer Ausführungsform ist in dem einen Verschlusselement eine Feststelleinrichtung für das Stauband vorgesehen. Das bewegliche Verschlusselement ist mit dem festen Verschlusselement magnetisch verbindbar.

Für eine weitere bevorzugte Ausführungsform ist das eine Verschlusselement auf einer dem anderen Verschlusselement zugewandten Seite an mindestens einer, bevorzugt mindestens zwei Außenkanten mit Fasen und Nuten und das andere Verschlusselement an mindestens einer, bevorzugt mindestens zwei Außenkanten mit Stegen und Federn versehen und beide Verschlusselemente sind an den Außenkanten im geschlossenen Zustand bündig zueinander ausgeführt. Die Fasen des einen Verschlusselements korrespondieren mit den Stegen des anderen Verschlusselements und die Nuten des einen Verschlusselements korrespondieren mit den Federn des anderen Verschlusselements.

Für eine weitere Ausführungsform ist eine Sensorik zur Ermittlung von Vitalparametern, wie beispielsweise Herzfrequenz/Puls, Blutdruck, Blutsauerstoffgehalt, und/oder einem Aktivitäts-Reinigungszustand in dem Stauband, wie beispielsweise Status durch Magnetsensor: auf/zu, Nähe zum Reinigungsort, Dehnungsmessstreifen, und/oder dem Verschluss, hier können beispielsweise ein Magnetsensor, Licht-, Schall-, Dehnungssensor zur Anwendung kommen, integriert.

Eine weitere Ausführungsform integriert im Stauband und/oder im Verschluss eine Sende- und Empfangseinheit, welche eine Weiterleitung von Informationen, welche beispielsweise von der Sensorik ermittelt wurden, ermöglicht.

Der erfindungsgemäße Verschluss für eine Vorrichtung zum Stauen von Gefäßen hat mindestens zwei Verschlusselemente, die lösbar miteinander verbunden sind. Das eine Verschlusselement ist fest mit einem Stauband und das andere Verschlusselement ist verschiebbar auf dem Stauband angeordnet. Die Verschlusselemente sind entsprechend des Anspruchs mit Fasen und Nuten und korrespondierenden Stegen und Federn versehen und sind an ihren Außenkanten bündig zueinander ausgeführt. Die Stege mit den Federn oder die Fasen mit den Nuten sind mit dem beweglichen oder dem festen Verschlusselement verbunden. Die Stege sind auf dem Verschlusselement in einem Winkel zum Verschlusselement angeordnet. Es ist vorstellbar, dass beide Stege mit einem anderen Winkel als 90 Grad in eine gleiche Richtung oder in eine gegensätzliche Richtung geneigt sind. Dann ist es erforderlich, dass die Fasen entsprechend gestaltet sind, so dass Stege und Fasen und Nuten und Federn ineinandergreifen können. Der Winkel zwischen den Stegen und den Verschlusselementen sollte sinnvoller Weise zwischen 30 und 150 Grad liegen, bevorzugt bei ca. 80 Grad. Die lösbare Verbindung kann beispielsweise durch eine magnetische Verriegelung erfolgen.

In einer Ausführungsform sind die Stege in Richtung des gegenüberliegenden Verschlusselements bogenförmig (gekrümmt) ausgeführt und die Fasen sind korrespondierend zu den Stegen gestaltet.

Für eine bevorzugte Ausführungsform sind die Stege auf dem Verschlusselement etwas breiter in der Art, dass die Stege eine Basis und eine Spitze aufweisen, wobei die Basis mindestens 0,1 mm, bevorzugt 0,5 mm bis 5 mm breiter als die Spitze ist, und die Fasen korrespondierend zu den Stegen gestaltet sind, so dass ein einfach gleitendes Zusammenspiel beider Verschlusselemente zueinander gewährleistet ist, welches durch mindestens einen flexiblen Steg noch verbessert werden kann.

Mit den Verschlusselementen sind Magnete verbunden, welche mit ihren Magnetfeldern zwischen beiden Verschlusselementen wirken und welche durch ihre gegensätzliche Polung die Verschlusselemente ineinander ziehen, wodurch eine Verriegelung erfolgt. Die magnetische Kraft der verbundenen Verschlusselemente kann zusätzlich den Arretierungsmechanismus initiieren und das Band bei geschlossenem Verschluss (in einer Richtung) sperren. Ein abstoßendes Magnetfeld kann an der vierten Außenkante des ersten Verschlusselements wirken, wodurch ein Lösen beider Verschlusselemente voneinander beschleunigt wird. Weiterhin kann das Magnetfeld auch elektronisch betrieben werden, um beispielsweise die Feldstärke des Magnetfeldes gezielt zu regeln oder die Sensorik einstellen zu können. So kann auch eine Abschaltung der Elektronik, beispielsweise nach Benutzung, ermöglicht werden.

Für eine besondere Ausführungsform ist innerhalb des einen Verschlusselements eine Feststelleinrichtung mit einem Oberflächenrelief angeordnet, welche ein passgenaues Gegenstück zu einem Stauband ist, das mit einem korrespondierenden Oberflächenrelief versehen ist.

An dem Verschluss kann zusätzlich ein Hebel oder ein Rad angeordnet sein, um eine zusätzliche Spannung des Staubandes aufzubauen.

Ebenso kann eine Blutdruckmanschette eine Vorrichtung zum Stauen von Gefäßen entsprechend der erfindungsgemäßen Lösung verwenden und mit einem erfindungsgemäßen Verschluss verbunden sein.

Die erfindungsgemäße Vorrichtung zum Stauen von Gefäßen ermöglicht einen einfacheren Nutzungsablauf bei verbesserten Hygieneeigenschaften. Die verbesserte Nutzbarkeit für den Anwender wird durch eine einhändige Bedienung im ganzen Prozess ermöglicht. Durch die Magnetkraft in dem Verschluss wird der gesamte Verschlussmechanismus quasi selbstständig ausgeführt. Der Verschluss findet sich sozusagen von allein. Dabei können die Magnete im Verschluss eingearbeitet sein, um nicht die Oberfläche des Verschlusses zu bilden.

Das Öffnen des Verschlusses beginnt durch eine quer zum Stauband gerichtete Bewegung, beispielsweise eine simple Wischbewegung des Daumens (wie beim Entsperren eines Smartphones). Durch die Stegform kann der Öffnungsvorgang ebenfalls in einer kurvenförmigen Bewegung ausgebildet werden. Nach Überwindung des magnetischen Potentialmaximums, das der Sicherung des Verschlusses dient, können abstoßende Kräfte den Öffnungsvorgang des Verschlusses unterstützen.

Durch die glatten Teile des Staubandes und des Verschlusses wird die schnellere Reinigung und die Befreiung von auf dem Gefäßstauer befindlichen Keimen maßgeblich vereinfacht und verbessert. Die Entstehung eines Keimreservoirs wird durch die Verlagerung der beweglichen äußeren Verschlussteile ins Innere verhindert, so dass es keine prominenten/unebenen Anlagerungsorte für Keime gibt. Eine Vorrichtung im Innenbereich kann zusätzlich dafür sorgen, dass sich der Gefäßstauer auch ohne äußere Maßnahmen selbst reinigt. Eine Ausführungsform wäre eine Umlenkrolle aus ionisch-bakteriziden Materialien. Ein Autoklavieren oder andere technische Sterilisationsmaßnahmen sind nicht mehr notwendig.

Außerdem wird es durch die Integration entsprechender Elektronik möglich, Vitalfunktionen des Patienten sowie den Reinigungsstatus des Gefäßstauers zu erfassen und beispielsweise per Funk an ein zentrales System zu übertragen.

### Ausführung der Erfindung

Die Erfindung wird anhand von Ausführungsbeispielen näher erläutert. Hierzu zeigen
- Figur 1: eine erfindungsgemäße Vorrichtung zum Stauen von Gefäßen in perspektivischer Ansicht,
- Figur 2: die Vorrichtung zum Stauen von Gefäßen in einer anderen perspektivischen Ansicht,
- Figur 3: eine Seitenansicht zu Figur 1,
- Figur 4: ein Querschnitt eines geschlossenen Verschlusses,
- Figur 5: eine Blutdruckmanschette mit Vorrichtung zum Stauen von Gefäßen,
- Figur 6: Verschluss in perspektivischer Ansicht, und
- Figur 7: Querschnitt des Verschlusses.

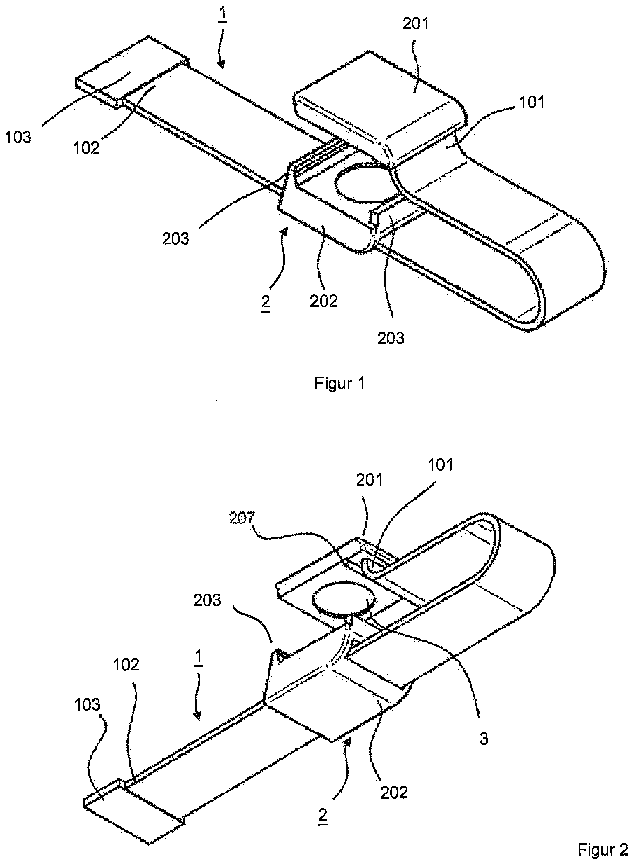

Die Figuren 1 bis 3 zeigen die erfindungsgemäße Vorrichtung zum Stauen von Gefä-ßen in verschiedenen perspektivischen Ansichten und in einer Seitenansicht mit einem geöffneten Verschluss.

Die erfindungsgemäße Vorrichtung zum Stauen von Gefäßen besteht aus einem elastischen Stauband 1 mit glatter Oberfläche, beispielsweise aus einem textilfreien Silikonmaterial. Das Stauband 1 ist mit einem Verschluss 2 verbunden, so dass es zu einem geschlossenen Rundband verbunden werden kann. Der Verschluss 2 besteht aus mindestens zwei Verschlusselementen 201 und 202, die mittels einer magnetischen Verriegelung lösbar verbunden sind. Weiterhin ist der Verschluss 2 im geschlossenen Zustand, außer der abschließenden Kante zwischen den Verschlusselementen, ein glatter, quaderförmiger oder länglicher Körper mit vorzugsweise gerundeten Kanten, welcher außer den Zuführungen für das Stauband 1 keine weiteren äußeren Öffnungen oder Absätze aufweist, da die Kanten zwischen einem ersten, festen Verschlusselement 201 und einem zweiten, beweglichen Verschlusselement 202 bündig abschließen. Weitere abschließende Kanten, z.B. zwischen fertigungstechnisch bedingter Ober- und Unterschale, sind als Öffnungen vernachlässigbar.

An einem ersten Ende 101 des Staubandes ist das erste, obere Verschlusselement 201 in der Art angeordnet, dass das Stauband 1 in dieses Verschlusselement 201 eingefädelt ist und beispielsweise durch Kleben, Druck, Schrauben oder magnetisch gesichert wird, so dass das Stauband 1 fest mit dem Verschlusselement 201 verbunden ist. Das zweite Ende 102 des Staubandes 1 ist in einer dafür vorgesehenen, durchgehenden Öffnung durch das zweite, untere, verschiebbare Verschlusselement 202 hindurchgeführt und an seinem Ende 102 durch einen Stoppmechanismus 103 begrenzt, damit das auf dem glatten elastischen Stauband 1 verschiebbare Verschlusselement 202 nicht von dem Stauband 1 gleiten kann. In dem zweiten Verschlusselement 202 ist eine Feststelleinrichtung 205, zum Beispiel in Form einer Gleitschiene, mit einem Arretierungsmechanismus, beispielsweise einer Wippe, eines Keils, einer Feder, einer aufgerauten Oberfläche oder einer Druckrolle, für das Stauband 1 vorgesehen. Das bewegliche Verschlusselement 202 ist magnetisch mit dem festen Verschlusselement 201 verbindbar und verriegelt sich automatisch mechanisch. Die Feststelleinrichtung wird nach der Verbindung beider Verschlusselemente 201, 202 miteinander automatisch durch den Spannvorgang um einen Gegenstand betätigt.

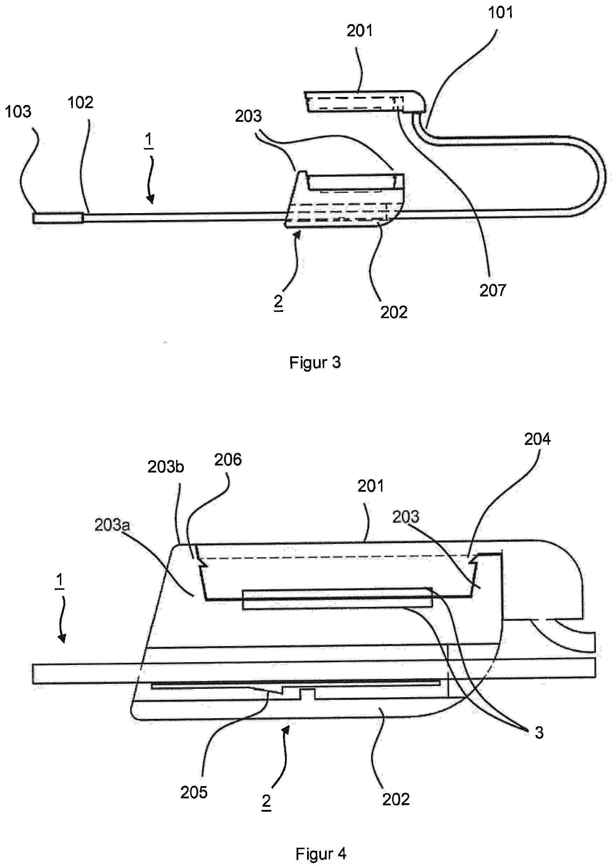

Für die magnetische Verbindung beider Verschlusselemente 201, 202 wird ein magnetischer Verschlussmechanismus angewendet. So wird durch die besondere, erfindungsgemäße Gestaltung des Verschlusses 2 ein einhändiges Schließen, Spannen und Öffnen des Staubandes 1 ermöglicht. In Figur 4 wird ein geschlossener Verschluss 2 gezeigt. In jedem Verschlussteil 201, 202 ist mittig und zueinander gerichtet ein Magnet 3 eingearbeitet.

Eines der beiden Verschlusselemente 201, 202, beispielsweise das zweite, untere Verschlusselement 202, ist auf der dem anderen Verschlusselement 201 zugewandten Seite an mehreren, z.B. zwei Außenkanten mit Stegen 203 quer zum Stauband 1 versehen. Die Stege 203 sind an ihrem oberen Ende, ins Innere des Verschlusses zeigend, mit Federn 206 versehen. Entsprechend ist das erste Verschlusselement 201 so gestaltet, dass beide Verschlusselemente 201, 202 ineinandergreifen können. Das erste Verschlusselement 201 ist dazu an beispielsweise zwei Außenkanten mit einer vorderen und einer hinteren Fase 207 versehen. An zwei, nach außen zeigenden Flächen der Fasen 207 sind Nuten 204 eingearbeitet, welche mit den Federn 206 korrespondierenden. Die Federn 206 greifen in die Nuten 204 ein und ermöglichen so eine zusätzliche Sicherung. Die dritte und vierte Außenkante, vorzugsweise in Längsrichtung zum Stauband 1, ist mit dem jeweils anderen Verschlusselement 201, 202 bündig ausgestaltet. Die Stege 203, und die Federn 206 können mit dem beweglichen oder dem festen Verschlusselement 201, 202 verbunden sein. Das gleiche gilt für die Nuten 204 und die Fasen 207 in korrespondierender Weise zu den Federn 206 und Stegen 203. Die Stege 203 sind auf dem Verschlusselement 201, 202 in diesem Ausführungsbeispiel in einem rechten Winkel zum Verschlusselement 201, 202 angeordnet. Der Winkel zwischen den Stegen 203 und den Verschlusselementen 201, 202 sollte sinnvoller Weise zwischen 30 und 150 Grad liegen, bevorzugt bei ca. 80 Grad. Sie können für eine bessere Handhabbarkeit des Verschlusses 2 zum Verschlusselement 201, 202 hin etwas breiter in der Art sein, dass die Stege 203 auf dem Verschlusselement 201, 202 eine Basis 203a und eine Spitze 203b aufweisen, wobei die Basis 203a mindestens 0,1 mm, bevorzugt 0,5 mm bis 5 mm breiter als die Spitze 203b ist. Die Fasen 207 sind entsprechend gestaltet, so dass in jedem Fall ein einfach gleitendes Zusammenspiel beider Verschlusselemente 201, 202 zueinander gewährleistet ist. Durch die sich im Innern des Verschlusses 2 befindlichen Magnete 3 finden sich beide Verschlusselemente 201, 202 selbst, wenn sie in den Wirkungskreis des Magnetfeldes kommen. Es wird eine einhändige Bedienung (Anlegen, Spannen, Lockern und Lösen) des Staubandes 1 ermöglicht. Für das Lösen beider Verschlusselemente 201, 202 voneinander wird das obere Verschlusselement 201, beispielsweise mit dem Daumen, in die Richtung geschoben, in der auf dem unteren Verschlusselement 202 kein Steg 203 ist, wodurch mehrere Richtungen denkbar sind. Die Bewegung erfolgt soweit, bis die anziehende Wirkung der Magnete 3 nachlässt und sich beide Verschlusselemente 201, 202 durch die (mögliche) einsetzende abstoßende Wirkung voneinander trennen.

Das Stauband 1 und optional der Verschluss 2 sind durch Fluorierung von dessen Oberfläche / von deren Oberflächen mit einer glättenden Beschichtung versehen. Die außerordentliche Glattheit des Staubandes und/oder des Verschlusses wird durch die besondere Beschichtung erreicht. Bei der Fluorierung werden Wasserstoffatome aus den Seitenketten herausgelöst und durch Fluor ersetzt, wodurch sich die Oberflächeneigenschaften ändern. Zum einen werden die Haft- und Gleitreibung sowie die Klebrigkeit deutlich verringert, zum anderen wird die Benetzbarkeit erhöht, wodurch polare Flüssigkeiten, wie bei einer Flüssigdesinfektion, einen besonderen Oberflächenfilm bilden und nicht mehr dazu neigen durch die Eigenpolarität lokale Tropfen auszubilden. So entstehen bessere Gleiteigenschaften mit fehlerfreien Flüssigkeitsfilmen, was bei dem Gefäßstauer zu besseren Anwendung auf der Haut und besseren Reinigbarkeit durch Benetzen der gesamten Oberfläche und damit der Befreiung von Keimen an jeder Stelle führt. Hierdurch wird verhindert, dass Keime nach der Anwendung des Gefäßstauers auf dem Gerät zurückbleiben und ein Infektionsrisiko für den nächsten Patienten darstellen. Geprüft werden kann diese Beschichtung durch einen Kipptest, bei dem das beschichtete Material und das unbeschichtete Material auf einer Ebene liegen die sich bei 0° befindet und fließend/kontinuierlich bis zu 100° geneigt werden kann. Das hier beschriebene Stauband erreicht zu Zwecken der Hygiene weniger als 35°, wenn es zu gleiten beginnt.

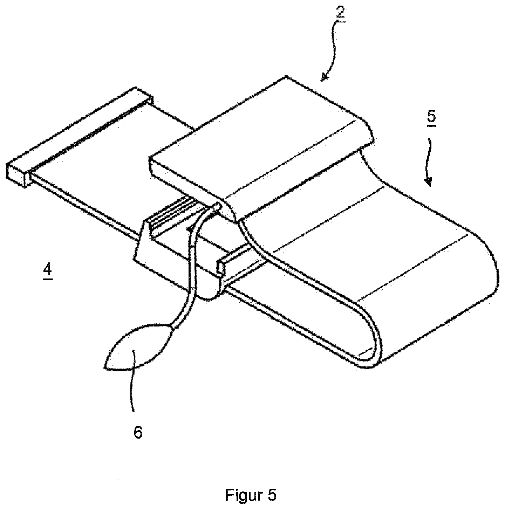

Figur 5 zeigt eine Blutdruckmanschette 4 mit einer Vorrichtung zum Stauen von Gefäßen als ein weiteres Ausführungsbeispiel. Dazu wird mit dem oben ausführlich beschriebenen Verschluss 2, welcher für diese Anwendung größer als für das Stauband 1 dimensioniert sein kann, ein glattes Band 5 in beschriebener Weise verbunden. Die variable Druckeinstellung kann, wie nach dem Stand der Technik üblich, durch unterschiedliche Mechanismen erfolgen, als gängigste sei hier nur der pneumatische Druckanstieg mittels Pumpe genannt.

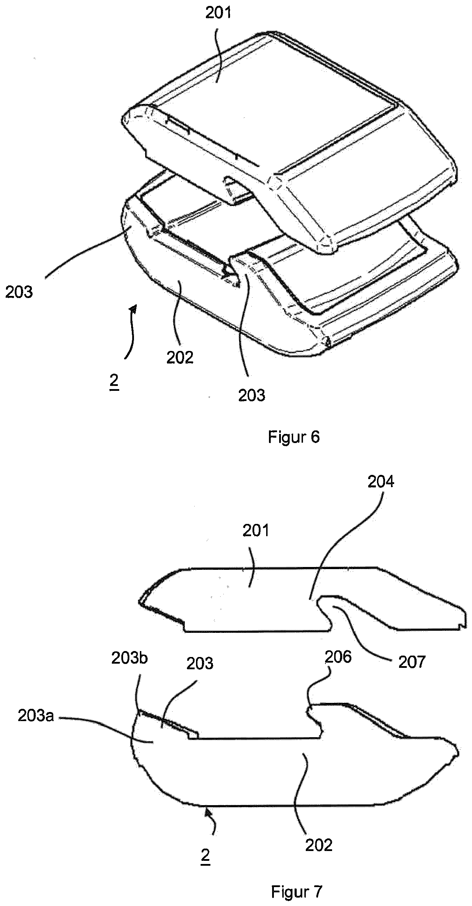

Figur 6 und 7 zeigt den Verschluss 2 mit bogenförmigen Stegen 203, welche in entsprechend geformte Nuten 204 eingreifen, wodurch die Verschlusselemente 201, 202 durch die Kraftübertragung bei Zug besser ineinander gehalten werden. Die Magnete 3 sind in dieser Ausführungsform innerhalb des Verschlusses 2 lokalisiert. Die Rundung der ineinandergreifenden bogenförmigen Stege 203 trägt dazu bei, dass die Scherkräfte in einem größeren Kreissegment wirken als bei ausschließlich gerader Kantenführung. Hierdurch wird ein sicherer Halt der Verschlusselemente 201, 202 zueinander gewährleistet und die Haltekräfte des magnetischen Potentialmaximums werden unterstützt.

Weiterhin ist denkbar den Gefäßstauer ohne Magneten abzubilden, um eine Benutzung in der Nähe eines Magnetresonanztomographen zu ermöglichen. Dafür können auch Verschlüsse 2 dienen, die nur zum Festziehen und Lösen gedacht sind, und möglicherweise nicht einmal zum Öffnen sind, sondern nur über den Arm gestreift werden, aber die hygienischen Eigenschaften der benannten Erfindung tragen und eine entsprechende Gleitbeschichtung haben.

Weiterhin ist denkbar in den oben beschriebenen Gefäßstauer Sensoren zu integrieren, wie z.B. Dehnungsmessstreifen oder Fasern, die Längenänderungen im Stauband 1 oder im Band 5 resistiv wahrnehmen. Die Messung des Blutdrucks oder anderer Vitalparameter kann mit Hilfe des bereits bestehenden Spannmechanismus erfolgen, indem die nötige Vorspannung durch die erfindungsgemäße Vorrichtung zum Stauen von Gefäßen erzeugt wird. Die Vorspannung muss dann nicht mehr durch Pumpen variiert werden. Durch eine im Stauband 1 und/oder im Verschluss 2 integrierte Sensorik zur Ermittlung von Vitalparametern kann u.a. der Blutdruck erfasst werden, was ein neuartiges Messverfahren des Blutdrucks darstellt. Ebenso kann eine entsprechende Sensorik in dem Gefäßstauer bzw. der Blutdruckmanschette dazu beitragen, den Reinigungsstatus zu erfassen.

Auch ist es denkbar, dass im Stauband 1 und/oder im Verschluss 2 eine Sende- und Empfangseinheit integriert ist, welche die Weiterleitung von Informationen ermöglicht. Durch die Kommunikation mit einer entsprechenden Basisstation kann der hygienische Zustand des Gefäßstauers und die Vitalparameter übermittelt und dokumentiert werden. Das wiederum trägt zur Einhaltung von Hygienerichtlinien bei. Dazu kann in dem Stauband 1 oder dem Verschluss 2 eine Anzeigeeinheit integriert sein.

Durch veränderte Eigenschaften des Staubandes 1, beispielsweise in Form eines (gezackten) Oberflächenreliefs und geringerer Dehnbarkeit kann ein höherer Staudruck aufgebaut werden, um speziell in Notfallsituationen den kompletten Blutfluss der Arterien und Venen zu stoppen. Dazu muss innerhalb des zweiten Verschlusselements 202 die Gleitschiene der Feststelleinrichtung 205 mit einem dem Oberflächenrelief entsprechenden passgenauen Gegenstück angeordnet sein. Eine zusätzliche Spannung des Staubandes 1 kann nach der Arretierung beispielsweise über einen Hebel oder ein Rad an dem Verschluss 2 erfolgen. Die Hygiene spielt in diesen Extremsituationen eine untergeordnete Rolle. Maßgeblich ist, dass die einhändige Bedienung durch den selbstfindenden Magnetverschluss einen enormen Überlebensvorteil darstellt.

Die Vorrichtung zum Stauen von Gefäßen und/oder der Verschluss 2 für die Vorrichtung zum Stauen von Gefäßen und/oder die Blutdruckmanschette 4-wird durch folgende Schritte gereinigt:
- Auftragen eines Reinigungs- oder Desinfektionsmittels mithilfe eines Desinfektionsmittelspenders. Der Desinfektionsmittelspender kann dazu fest an einer Wand montiert sein oder es kann eine mobile Flasche mit Desinfektionsmittel genutzt werden.
- Alternativ kann ein Eintauchen in ein Reinigungs- oder Desinfektionsmittelbad vorgenommen werden.
- Oder es erfolgt ein Behandeln mit einem mit Reinigungs- oder Desinfektionsmittel getränkten Tuch,
- Die Desinfektionsflüssigkeit sollte mindestens 1 s bis maximal 10 min, bevorzugt 30 s, einwirken.
- Abschließend werden Desinfektionsflüssigkeitsreste, welche bei einer der drei Auftragsmethoden noch vorhanden sind, durch Abwischen, beispielsweise mit einem Einmaltuch, entfernt.

Zur Anwendung für das Verfahren kann eine stationäre oder portable, beispielsweise auf einem Tablett angeordnete, Reinigungsvorrichtung kommen, welche hier nicht weiter dargestellt werden soll. Durch diese Vorrichtung wird der Gefäßstauer transportiert, indem beispielsweise Walzen oder Rollen den Vortrieb leisten. Die Walzen oder Rollen sind mit Desinfektionsmittel getränkten Schwämmen versehen. Durch ein zweites Paar von Schwämmen, Walzen oder Rollen werden Flüssigkeitsreste abgetragen. Der Transport kann kontinuierlich oder diskontinuierlich erfolgen. Eine andere Möglichkeit besteht darin, dass das Stauband 1 sowie der Verschluss 2 oder auch die Blutdruckmanschette 4 in eine dafür vorgesehene Aussparung gelegt werden, welche aus beispielsweise nachgebenden Schwämmen besteht. Entweder werden auch hier die Walzen oder Rollen über den Gefäßstauer bewegt oder der Gefäßstauer wird durch die Vorrichtung gezogen.

Da es sich bei den vorhergehenden, detailliert beschriebenen Vorrichtungen zum Stauen von Gefäßen um Ausführungsbeispiele handelt, können sie in üblicher Weise vom Fachmann in einem weiten Umfang modifiziert werden, ohne den Bereich der Erfindung zu verlassen. Insbesondere können auch die konkreten Ausgestaltungen des Verschlusses 2 und der Verschlusselemente 201, 202 in anderer Form als in der hier beschriebenen folgen. Ebenso kann das Stauband 1, insbesondere die Blutdruckmanschette 4 in einer anderen Form ausgestaltet werden, wenn dies aus Platzgründen, designerischen oder technologischen Gründen notwendig ist. Weiter schließt die Verwendung der unbestimmten Artikel "ein" bzw. "eine" nicht aus, dass die betreffenden Merkmale auch mehrfach vorhanden sein können.

### Bezugszeichen

- 1: Stauband
101 erstes Ende des Staubandes 1
102 zweites Ende des Staubandes 1
103 Stoppmechanismus
- 2: Verschluss
201 erstes Verschlusselement
202 zweites Verschlusselement
203 Steg
203a Basis des Stegs
203b Spitze des Stegs
204 Nute
205 Feststelleinrichtung
206 Feder
207 Fasen
- 3: Magnet
- 4: Blutdruckmanschette
- 5: Band
- 6: Pumpe

## Patentansprüche

1. Stauband (1) für eine hygienische Vorrichtung zum Stauen von Gefäßen, welches ausgebildet ist, mit Hilfe eines Verschlusses zu einem geschlossenen Rundband verbunden zu werden, wobei
das Stauband aus einem textilfreien Material besteht und das Stauband durch Fluorierung von dessen Oberfläche mit einer Gleitbeschichtung versehen ist.

2. Stauband (1) nach Anspruch 1, wobei
die Gleitbeschichtung die Oberfläche des Staubandes (1) versiegelt.

3. Stauband (1) nach einem der vorhergehenden Ansprüche, wobei
das Stauband (1) aus einem elastischen Material mit einer geschlossenen Oberfläche gefertigt ist.

4. Stauband (1) nach Anspruch 3, wobei
es sich bei dem elastischen Material mit geschlossener Oberfläche um Silikon oder Latex handelt.

5. Stauband (1) nach einem der vorhergehenden Ansprüche, wobei
das Stauband (1) eine Breite von 0,5 bis 50 cm, insbesondere von 1 bis 10 cm oder 7 bis 30 cm, aufweist.

6. Stauband (1) nach einem der vorhergehenden Ansprüche, wobei
das Stauband (1) mit einem Oberflächenrelief versehen ist.

7. System umfassend
ein Stauband (1) nach einem der vorhergehenden Ansprüche und ein erstes Verschlusselement (201), wobei
ein Ende (101) des Staubands (1) fest mit dem ersten Verschlusselement (201) verbunden ist.

8. System nach Anspruch 7, ferner umfassend
ein zweites Verschlusselement (202), das gleitend verschiebbar auf dem Stauband (1) angeordnet ist.

9. System umfassend ein Stauband (1) nach einem der Ansprüche 1 bis 6 und ein erstes Verschlusselement (202), wobei
das erste Verschlusselement (202) gleitend verschiebbar auf dem Stauband (1) angeordnet ist.

10. System nach Anspruch 9, ferner umfassend
ein zweites Verschlusselement (201), wobei ein Ende (101) des Staubands (1) fest mit dem zweiten Verschlusselement (201) verbunden ist.

11. System nach einem der Ansprüche 7 bis 10, wobei
eine Sensorik zur Ermittlung von Vitalparametern und/oder eines Reinigungszustands in das Stauband (11) integriert ist.

12. System nach einem der Ansprüche 7 bis 11, wobei
in das Stauband (1) eine Sende- und/oder Empfangseinheit integriert ist, welche eine Weiterleitung von Informationen ermöglicht.

13. System nach einem der Ansprüche 7 bis 12, wobei das System als Blutdruckmanschette mit einer Breite des Staubands zwischen 7 und 30 cm ausgebildet ist.

14. Verfahren zum Reinigen eines Staubands (1) nach einem der Ansprüche 1 bis 6, wobei
eine Walze oder Rolle, welche Desinfektionsmittel aufgenommen hat, über das Stauband (1) bewegt wird.

15. Verwendung des Staubandes (1) nach einem der Ansprüche 1 bis 6 oder des Systems nach einem der Ansprüche 7 bis 13 für einen Gefäßstauer, vorzugsweise für eine Blutdruckmanschette oder für einen Venenstauer.

## Claims

1. A constricting belt (1) for a hygienic device for constricting vessels, which is configured to be connected by means of a lock to form a closed circular belt, wherein
the constricting belt consists of a textile-free material and the constricting belt is provided with an anti-friction coating by fluorination of its surface.

2. The constricting belt (1) according to claim 1, wherein
the anti-friction coating seals the surface of the constricting belt (1).

3. The constricting belt (1) according to any of the preceding claims, wherein
the constricting belt (1) is made of an elastic material with a closed surface.

4. The constricting belt (1) according to claim 3, wherein
the elastic material with the closed surface is silicone or latex.

5. The constricting belt (1) according to any of the preceding claims, wherein
the constricting belt (1) has a width of 0.5 to 50 cm, in particular of 1 to 10 cm or 7 to 30 cm.

6. The constricting belt (1) according to any of the preceding claims, wherein
the constricting belt (1) is provided with a surface relief.

7. A system comprising
a constricting belt (1) according to any of the preceding claims and a first lock element (201), wherein
one end (101) of the constricting belt (1) is fixedly connected to the first lock element (201).

8. The system according to claim 7, further comprising
a second lock element (202) slidably arranged on the constricting belt (1).

9. A system comprising
a constricting belt (1) according to any one of claims 1 to 6 and a first lock element (202), wherein
the first lock element (202) is slidably arranged on the constricting belt (1).

10. The system according to claim 9, further comprising
a second lock element (201), wherein one end (101) of the constricting belt (1) is fixedly connected to the second lock element (201).

11. The system according to any one of claims 7 to 10, wherein
a sensor system for determining at least one of vital parameters and a cleaning condition is integrated into the constricting belt (1).

12. The system according to any one of claims 7 to 11, wherein
at least one of a transmitting unit and a receiving unit is integrated into the constricting belt (1), which unit enables information to be forwarded.

13. The system according to any one of claims 7 to 12, wherein the system is configured as a blood pressure cuff with a width of the constricting belt between 7 and 30 cm.

14. A method for cleaning a constricting belt (1) according to any one of claims 1 to 6, wherein
a roller or a roll, which has received disinfectant, is moved over the constricting belt (1).

15. Use of the constricting belt (1) according to any one of claims 1 to 6 or of the system according to any one of claims 7 to 13 for a vascular tourniquet, preferably for a blood pressure cuff or for a venous tourniquet.

## Revendications

1. Bande de compression (1) pour dispositif d'hygiène permettant de comprimer des vaisseaux sanguins, laquelle bande est conçue pour être attachée à l'aide d'un fermoir de manière à former un rond fermé,
la bande de compression étant composée d'une matière non textile et dotée d'un revêtement lisse dont la surface est traitée par fluoration.

2. Bande de compression (1) selon la revendication 1,
la surface de la bande de compression (1) étant imperméabilisée par le revêtement lisse.

3. Bande de compression (1) selon l'une des revendications précédentes,
la bande de compression étant fabriquée à partir d'une matière élastique à surface fermée.

4. Bande de compression (1) selon la revendication 3,
la matière élastique à surface fermée étant constituée de silicone ou de latex.

5. Bande de compression (1) selon l'une des revendications précédentes,
cette bande de compression (1) présentant une largeur comprise entre 0,5 et 50 cm, plus particulièrement entre 1 à 10 cm ou entre 7 et 30 cm.

6. Bande de compression (1) selon l'une des revendications précédentes,
cette bande de compression (1) étant dotée d'un relief superficiel.

7. Système comportant
une bande de compression (1) selon l'une des revendications précédentes et un premier élément de fermeture (201),
une extrémité (101) de la bande de compression (1) étant reliée de manière fixe au premier élément de fermeture (201).

8. Système selon la revendication 7, comportant en outre
un deuxième élément de fermeture (202) disposé de manière à pouvoir coulisser sur la bande de compression (1),

9. Système comportant
une bande de compression (1) selon l'une des revendications 1 à 6 et
un premier élément de fermeture (202),
le premier élément de fermeture (202) étant disposé de manière à pouvoir coulisser sur la bande de compression (1).

10. Système selon la revendication 9, comportant en outre
un deuxième élément de fermeture (201), une extrémité (101) de la bande de compression (1) étant reliée de manière fixe au deuxième élément de fermeture (201).

11. Système selon l'une des revendications 7 à 10,
un système de capteurs servant à la détection de paramètres vitaux et/ou d'un état de nettoyage étant incorporé dans la bande de compression (1).

12. Système selon l'une des revendications 7 à 11,
une unité d'émission et/ou de réception étant incorporée dans la bande de compression (1), laquelle unité permet la transmission d'informations.

13. Système selon l'une des revendications 7 à 12,
ce système étant réalisé sous la forme d'un brassard de pression artérielle avec une largeur de bande de compression comprise entre 7 et 30 cm.

14. Procédé de nettoyage de la bande de compression (1) selon l'une des revendications 1 à 6,
un cylindre ou un rouleau contenant un produit de désinfection étant déplacé sur la bande de compression (1).

15. Utilisation de la bande de compression (1) selon l'une des revendications 1 à 6 ou du système selon l'une des revendications 7 à 13 pour un dispositif de compression, préférentiellement pour un brassard de pression artérielle ou pour un garrot médical.
